Europäisches Patentamt

European Patent Office

Office européen des brevets

(19)

(11) **EP 1 484 084 A1**

(12) **EUROPEAN PATENT APPLICATION**
published in accordance with Art. 158(3) EPC

(43) Date of publication:
**08.12.2004 Bulletin 2004/50**

(51) Int Cl.⁷: **A61N 1/30**, A61N 1/04

(21) Application number: **03703035.0**

(22) Date of filing: **23.01.2003**

(86) International application number:
**PCT/JP2003/000601**

(87) International publication number:
**WO 2003/061758 (31.07.2003 Gazette 2003/31)**

(84) Designated Contracting States:
**AT BE BG CH CY CZ DE DK EE ES FI FR GB GR HU IE IT LI LU MC NL PT SE SI SK TR**
Designated Extension States:
**AL LT LV MK RO**

(30) Priority: **24.01.2002 JP 2002015305**

(71) Applicants:
• **HISAMITSU PHARMACEUTICAL CO. INC.**
**Tosu-shi, Saga-ken 841-0017 (JP)**
• **Kyodo Printing Co., Ltd.**
**Tokyo 112-8501 (JP)**

(72) Inventors:
• **MORI, K.;**
**c/o Tsukuba Lab. Hisamitsu Pharma. Co.**
**Tsukuba-shi, Ibaraki 305-0856 (JP)**
• **MAEDA, H.;**
**c/o Tsukuba Lab. Hisamitsu Pharma. Co.**
**Tsukuba-shi, Ibaraki 305-0856 (JP)**
• **NISHI, Y.;**
**c/o Tsukuba Lab. Hisamitsu Pharma. Co.**
**Tsukuba-shi, Ibaraki 305-0856 (JP)**
• **ARIMOTO, T.;**
**c/o Tsukuba Lab. Hisamitsu Pharma.**
**Tsukuba-shi, Ibaraki 305-0856 (JP)**
• **HIGO, N.;**
**c/o Tsukuba Lab. of Hisamitsu Pharma Co.**
**Tsukuba-shi, Ibaraki 305-0856 (JP)**
• **SATO, S.;**
**c/o Tsukuba Lab. of Hisamitsu Pharma Co.**
**Tsukuba-shi, Ibaraki 305-0856 (JP)**
• **OGAWA, T.;c/o Kyodo Printing Co., Ltd.**
**Bunkyo-ku, Tokyo 112-8501 (JP)**
• **TAKAHASHI, S.;c/o Kyodo Printing Co., Ltd.**
**Bunkyo-ku, Tokyo 112-8501 (JP)**
• **FUCHITA, Yasushi; c/o Kyodo Printing Co., Ltd.**
**Bunkyo-ku, Tokyo 112-8501 (JP)**

(74) Representative: **Westendorp, Michael, Dr.**
**Patentanwälte**
**Splanemann Reitzner**
**Baronetzky Westendorp**
**Rumfordstrasse 7**
**80469 München (DE)**

(54) **ELECTRODE STRUCTURE**

(57) An electrode structure is provided which is capable of efficiently using a material that can be a non-polarized electrode component, allowing the material to fully demonstrate the effect as the non-polarized electrode and maintaining the performance.

This electrode structure comprises an insulating base(13), a polarized component layer (first layer) (12) laminated on the insulating base so as not to penetrate the insulating base, the polarized component layer comprising a conductive paste or metallic foil which is predominantly made of a component that can be a polarized electrode, and a non-polarized component layer (second layer) (11) provided on the polarized component layer, the non-polarized component layer comprising a conductive paste which is predominantly made of a component that can be a non-polarized electrode.

FIG. 1

EP 1 484 084 A1

**Description**

Technical Field

The present invention relates to an electrode structure applicable to a living body used in medical fields such as medical treatment and diagnostics. More specifically, the present invention relates to an apparatus for delivering physiologically active substances into the living body using electric energy and an electrode structure used for an apparatus for extracting diagnostic substances from the living body to the outside.

Background Art

Iontophoresis (Acta Dermatol venereol, Vol. 64, p 93, 1984) and electroporation (Japanese Patent Publication No. 3-502416, Proc. Natl. Acad. Sci. USA, Vol. 90, pp 10504 to 10508, 1993) are methods for delivering physiologically active substances from skin and mucous membranes to the interior of the living body using electric energy.

Furthermore, a method for extracting diagnostic substances from the living body and observing pathological conditions using the same principle as iontophoresis is also used (Nature Medicine Vol. 1, pp.1198 to 1201, 1995).

Electrodes are required for these apparatuses for delivering physiologically active substances and extracting diagnostic substances, etc., and non-polarized components are generally used for the electrodes. A non-polarized electrode has advantages such as not causing polarization between an electrode and solution or not causing any changes in pH, etc. , and, on the other hand, since the electrode itself is active and provokes a chemical reaction, the duration that the electrode can be energized depends on the amount of the electrode component. For example, when silver is used as an anode, silver acts as a non-polarized electrode, but in that case silver is ionized according to the following equation (1):

$$Ag \rightarrow Ag^+ + e^- \qquad (1)$$

The relationship between the amount of silver and current that can be passed therethrough follows Faraday's law and an amount of electricity of 1 faraday per 1 mol of silver can be energized.

On the other hand, for the electrode used for the above described apparatus, a conductive paste is used for ease of its manufacturing method and one made into a thin film by a printing technology is used. In the case of silver as an example, a conductive paste whose silver content is 90 to 95% is made into a thin film by a screen printing technology, etc. , and used as an electrode.

However, the problem is that the electrode produced using such a conductive paste does not follow Faraday's law and it is only approximately 1/2 to 1/10 of the amount of electricity calculated from Faraday's law and the content of the electrode that can be energized. For this reason, the paste component grows in thickness, causing problems like an increase in the manufacturing cost and cracking during molding.

There are prior arts on the electrode structure using both polarized components and non-polarized components such as technologies described in WO97/06848 and Japanese Patent Application Laid-Open No. 2000-176024.

The former (WO97/06848) arranges a polarized electrode and a non-polarized electrode on the same plane, energizes from only one side and thereby promotes an electrochemical reaction on the skin and promotes transmission of physiologically active substances. The latter (Japanese Patent Application Laid-Open No. 2000-176024) has carbon, etc., penetrating an insulating base, creates a terminal part from a surface opposite to the surface on which the carbon layer of the insulating base is formed, has a plurality of these electrode structures (small electrodes) insulated from one another and uses these electrode structures arranged on the same plane as one electrode. This electrode is successful in passing a current at a uniform electric density by energizing small electrodes one by one.

However, these examples have problems in which since the electrode is divided into a plurality of small electrodes its productivity is low, and in which since a plurality of terminals penetrates an insulating base the plurality of terminals need to be connected during energization.

Disclosure of Invention

It is an object of the present invention to solve the above described problems of the prior arts.

That is, it is an object of the present invention to provide an electrode structure capable of efficiently using (letting react) a material that can be a non-polarized electrode component such as a conductive paste, allowing the material to fully demonstrate the effect as the non-polarized electrode and maintaining the performance as the non-polarized electrode. To achieve this object, the following points (1) to (3) are particularly taken into consideration:

(1) Enabling energization for a long period without increasing the amount of components that can be non-polarized electrodes.

(2) Preventing loss of performance as non-polarized electrodes, that is, preventing any change in pH.

(3) Providing an electrode with a simple structure that can be manufactured using a simple method and used in a simple manner.

The present inventors researched to solve the above described problems and have succeeded in completing an invention having the following structure.

That is, the present invention is an electrode structure characterized by comprising a single electrode on an insulating base, the single electrode including both a component that can be a polarized electrode and a component that can be a non-polarized electrode. Furthermore, the present invention is an electrode structure comprising an insulating base, a polarized component layer (first layer) laminated on the insulating base so as not to penetrate the insulating base, the polarized component layer comprising a conductive paste or metallic foil which is predominantly made of a component that can be a polarized electrode, and a non-polarized component layer (second layer) provided on the polarized component layer, the non-polarized component layer comprising a conductive paste which is predominantly made of a component that can be a non-polarized electrode.

The present invention is the above described electrode structure characterized in that the component that can be a polarized electrode is made of one or more materials selected from carbon, platinum, gold, aluminum and titanium.

The present invention is the above described electrode structure characterized in that the component that can be a non-polarized electrode is made of one or more materials selected from silver, silver chloride, copper and copper chloride.

The present invention is the above described electrode structure characterized in that the area of the non-polarized component layer is 1 to 10 cm$^2$.

The present invention is the above described electrode structure characterized in that the thickness of the polarized component layer is 1 to 100 μm.

The present invention is an electrode structure characterized in that the thickness of the non-polarized component layer is 5 to 500 μm.

The present invention is an electrode structure comprising a conductive paste on an insulating base, the conductive paste being a mixture of a component that can be a polarized electrode and a component that can be a non-polarized electrode.

Brief Description of Drawings

Figure 1 illustrates two examples of an electrode structure of the present invention, wherein (a) is a cross-sectional view of a first example, (b) is a perspective view of the first example, (c) is a cross-sectional view of a second example and (d) is a perspective view of the second example;

Figure 2 illustrates an insulating base produced with an aluminum film coated (laminated) with polyethylene terephthalate; and

Figure 3 is a graph showing amounts of energization according to an Example of the present invention and a Comparative Example.

Best Mode for Carrying Out the Invention

The present invention will be explained in detail below. The electrode structure according to the present invention is characterized by comprising a single electrode including a component that can be a polarized electrode and a component that can be a non-polarized electrode as electrode components, and these components may be mixed into a single-layer electrode or arranged in a laminated structure with the non-polarized component electrode disposed on the polarized electrode.

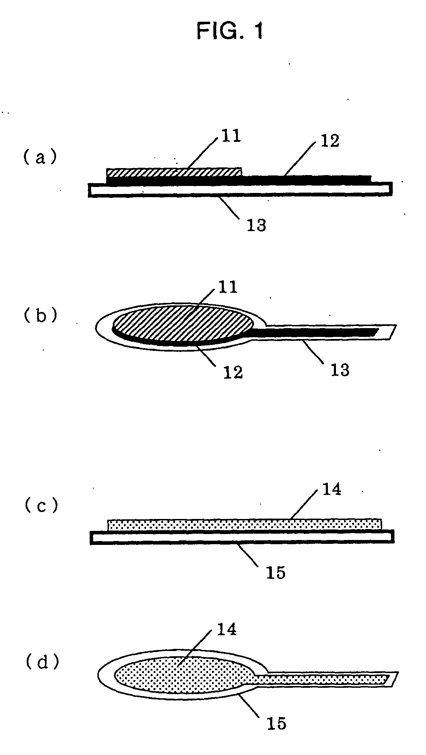

First, the case of the laminated structure will be explained. As shown in the cross-sectional view in (a) and the perspective view in (b) in Figure 1, the electrode structure of the present invention comprising an insulating base 13, a polarized component layer (first layer) 12 laminated on one side of the insulating base so as not to penetrate the insulating base, the polarized component layer comprising a conductive paste or metallic foil which is predominantly made of a component that can be a polarized electrode, and a non-polarized component layer (second layer) 11 provided on the polarized component layer (first layer), the non-polarized component layer comprising a conductive paste which is predominantly made of a component that can be a non-polarized electrode. Here, the protruding portion of the polarized component layer 12 is used as a connecting terminal with a power supply section which is not shown.

The above described electrode structure of the present invention has a structure having three main layers of the insulating base 13, polarized component layer 12 and non-polarized component layer 11 without requiring a plurality of terminals for connection with the power supply section or without penetrating the insulating base.

Then, the case of a single layer will be explained. As shown in the cross-sectional view in (c) and the perspective view in (d) in Figure 1, the electrode structure of the present invention is characterized by including a conductive paste 14 on an insulating base 15, the conductive paste being a mixture of a component that can be a polarized electrode and a component that can be a non-polarized electrode. Here, the protruding portion of the conductive paste 14 is used as a connecting terminal with a power supply section which is not shown.

The above described electrode structure of the present invention has a structure having two main layers of the insulating base 15 and the conductive paste 14 without requiring a plurality of terminals for connection with the power supply section or without penetrating the insulating base.

As prior arts related to the electrode structure using both a polarized component and non-polarized component as with the present invention, there are technologies described in the above described WO97/06848 and Japanese Patent Application Laid-Open No. 2000-176024, but these are different from the present invention and have the above described problems.

In order to solve the problems of these prior arts, the present invention extracts a terminal from the same plane (plane which is not opposite to the carbon layer of the insulating base) as that of the carbon layer of the insulating base, does not divide the electrode into a plurality of small electrodes, thus making it possible to improve productivity and avoid complexity when the product is in use.

That is, the electrode structure of the present invention improves the current flow and also takes into consideration productivity and ease of use.

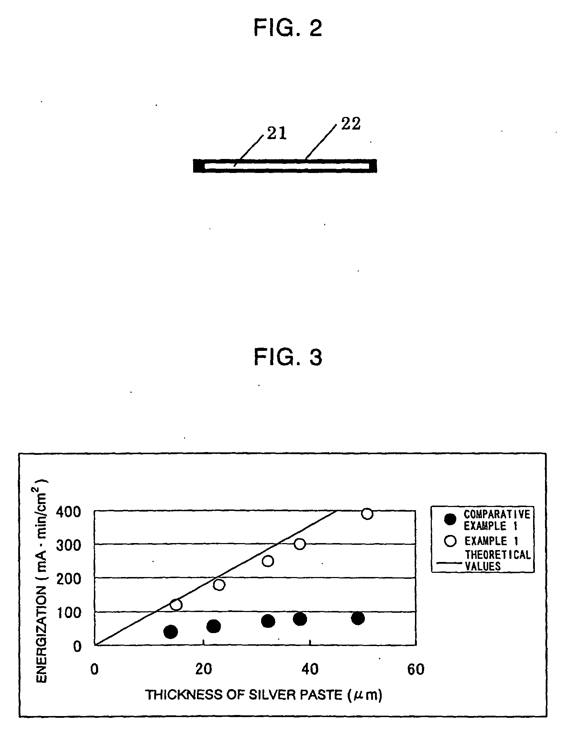

The material for the insulating bases 13 and 15 in the present invention is not particularly limited if it at least allows the polarized component layer 12 or conductive paste 14 to be laminated and has insulating properties, and can be polyethylene terephthalate, polyimide, polyamide, polypropylene, etc. These can also be used as a single film or a composite film and furthermore a conductive metal foil, etc., coated with an insulating film or laminated with an insulating film may also be used as the insulating base. An example of the latter is an aluminum foil 21 shown in Figure 2 coated with a polyethylene terephthalate 22.

The component that can be a polarized electrode in the present invention can be, for example, carbon, platinum, gold, aluminum, titanium and one or more materials thereof can be used, but the component is not limited to them. Among them, carbon is available on the market as a cheap conductive paste, and is particularly useful. The thickness of this polarized layer is not particularly limited, but is preferably 1 to 100 $\mu$m and 3 to 50 $\mu$m is especially preferable as the thickness, which provides flexibility and prevents damage such as cracking. The polarized electrode (polarized component layer) itself does not change and is acceptable as far as it allows a current to flow, but when the thickness of the polarized component layer is less than 1 $\mu$m, it is difficult to keep the uniformity with screen printing, failing to be shaped as expected, making it difficult to mold as a metal foil, preventing the polarized electrode from being formed as a uniform flat plane. Furthermore, when the thickness exceeds 100 $\mu$m, one produced with a conductive paste, etc., becomes brittle and is easily broken.

Furthermore, components that can be non-polarized electrodes in the present invention can be silver, copper, etc., on the anode side and a compound of silver/ silver chloride, etc. , predominantly made of silver chloride, copper chloride on the cathode side, and one or more materials thereof can be used, but components are not limited to them. Among them, silver (anode side) and silver/silver chloride (cathode side) are particularly preferable. The thickness of this non-polarized component layer is preferably 5 to 500 $\mu$m though it depends on the total amount of electricity energized and when flexibility of the electrode is taken into consideration, 10 to 400 $\mu$m is more preferable and approximately 25 to 100 $\mu$m is particularly preferable. Since the non-polarized electrode itself changes, if the thickness of the non-polarized component layer is less than 5 $\mu$m, it is difficult to keep uniformity and if there is even one thin part, that part changes through energization causing insulation, etc. On the other hand, when the thickness exceeds 500 $\mu$m, if produced with a conductive paste such as silver and silver/silver chloride, it becomes brittle and easily broken.

The area of the non-polarized electrode (non-polarized component layer) of the present invention is preferably 1 to 10 cm$^2$. When this area falls below 1 cm$^2$, it is difficult to create the electrode and when the area exceeds 10 cm$^2$, it is too big for the shape of formulation and hard to be pasted.

The electrode structure of the present invention can be manufactured by laminating the above described insulating base, polarized component layer and non-polarized component layer using an established method or by disposing a single-layer conductive paste on an insulating base, the single-layer conductive paste being a mixture of the polarized component and non-polarized component.

The electrode of the present invention can be used for any purpose when the electrode is at least pasted to a living body. For example, the electrode according to the present invention can be used as a cardiogram for diagnostics, an apparatus for non-invasively extracting diagnostic substances from a living body by passing a current as represented

## EP 1 484 084 A1

by an electrode described in GlucoWatch (Publication No. 10-505761 of Japanese Patent Application) or as an electrode of electroporation or iontophoresis for administering chemicals or physiologically active substances. When used as an electrode for iontophoresis, this electrode is applicable to the electrode structure described in Publication No. 11-54855 of Japanese Patent Application and Publication No. 10-234864 of Japanese Patent Application, etc.

(Example)

An Example of energization using the electrode according to the present invention and a Comparative Example of energization using a prior art will be shown below.

(Example 1)

Production of electrode of the present invention: A polyethylene terephthalate film was used as the insulating base 13 and a conductive paste predominantly made of carbon was applied onto this film to 10 μm in thickness (first layer 12). Furthermore, a conductive paste predominantly made of silver was applied onto the carbon to several types of thickness (second layer 11). The thickness of the silver paste after drying was 15, 23, 32, 38 and 51 μm, respectively.

Production of reference electrode: Two silver plates containing silver of purity greater than 99.99% were prepared, used as an anode and cathode, and energized in a physiological salt solution at 1 mA/cm$^2$ for six hours. At this time, the reacting electrode (silver/silver chloride electrode) on the anode side was used as a reference electrode.

Measurement of amount of energization: The electrode of the present invention was used as an anode and the reference electrode was used as a cathode, these electrodes were energized at 0.05 mA/cm$^2$ and energization was continued while measuring their voltages. Energization was stopped when the voltage reached a value, which exceeded the initial voltage by 1 voltage or more, and the point was regarded as the energization completion time. The amount of energization per unit area was calculated from the following equation:

$$\text{Amount of energization per unit area} = 0.05 \times \text{energization}$$

$$\text{completion time (minutes)} \qquad (2)$$

(Comparative Example 1)

Production of electrode: A polyethylene terephthalate film was used as the insulating base and a conductive paste predominantly made of silver was applied onto this film to several types of thickness. The thickness of the silver paste after drying was 14, 22, 32, 38 and 49 μm, respectively.

Reference electrode: Produced in the same manner of the reference electrode in Example 1.

Measurement of amount of energization: The electrode of Comparative Example 1 was used as an anode and the reference electrode was used as a cathode and these electrodes were energized and the amounts of energization were measured in the same manner of Example 1.

The results of Example 1 and Comparative Example 1 are shown in Figure 3. Theoretical values are also shown in Figure 3.

As is evident from Figure 3, while an amount of energization substantially close to the theoretical value is shown in Example 1, only approximately 18 to 33% of silver was used in Comparative Example.

That is, according to the electrode of the present invention, it has been confirmed that the efficiency of utilization of silver, which is a non-polarized component, is improved and the amount of energization is increased.

Industrial Applicability

The electrode structure of the present invention makes it possible to efficiently use a material that can be a non-polarized electrode such as a conductive paste, allow the material to fully demonstrate the effect as the non-polarized electrode and maintain the performance.

**Claims**

1. An electrode structure comprising a single electrode on an insulating base, the single electrode including both a component that can be a polarized electrode and a component that can be a non-polarized electrode.

2. An electrode structure comprising:

   an insulating base;
   a polarized component layer (first layer) laminated on the insulating base so as not to penetrate the insulating base, the polarized component layer comprising a conductive paste or metallic foil which is predominantly made of a component that can be a polarized electrode; and
   a non-polarized component layer (second layer) provided on the polarized component layer, the non-polarized component layer comprising a conductive paste which is predominantly made of a component that can be a non-polarized electrode.

3. The electrode structure according to claim 2, wherein the component that can be a polarized electrode is made of one or more materials selected from carbon, platinum, gold, aluminum and titanium.

4. The electrode structure according to claim 2, wherein the component that can be a non-polarized electrode is made of one or more materials selected from silver, silver chloride, copper and copper chloride.

5. The electrode structure according to claim 2, wherein the area of the non-polarized component layer is 1 to 10 cm$^2$.

6. The electrode structure according to claim 2, wherein the thickness of the polarized component layer is 1 to 100 $\mu$m.

7. The electrode structure according to claim 2, wherein the thickness of the non-polarized component layer is 5 to 500 $\mu$m.

8. An electrode structure comprising a conductive paste on an insulating base, the conductive paste being a mixture of a component that can be a polarized electrode and a component that can be a non-polarized electrode.

# FIG. 1

( a )

( b )

( c )

( d )

# FIG. 2

# FIG. 3

**INTERNATIONAL SEARCH REPORT**

| International application No. |
| --- |
| PCT/JP03/00601 |

**A. CLASSIFICATION OF SUBJECT MATTER**
    Int.Cl$^7$ A61N1/30, 1/04

According to International Patent Classification (IPC) or to both national classification and IPC

**B. FIELDS SEARCHED**

Minimum documentation searched (classification system followed by classification symbols)
    Int.Cl$^7$ A61N1/30, 1/04

Documentation searched other than minimum documentation to the extent that such documents are included in the fields searched
    Jitsuyo Shinan Koho          1922–1996   Toroku Jitsuyo Shinan Koho   1994–2003
    Kokai Jitsuyo Shinan Koho    1971–2003   Jitsuyo Shinan Toroku Koho   1996–2003

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>A | WO 97/06848 A1 (Hisamitsu Pharmaceutical Co., Inc.),<br>27 February, 1997 (27.02.97),<br>Full text; Figs. 1 to 6<br>Full text; Figs. 1 to 6<br>& JP 8-743 A          & EP 845280 A1 | 1,3-7<br>2,8 |
| A | WO 97/07853 A1 (Hisamitsu Pharmaceutical Co., Inc.),<br>06 March, 1997 (06.03.97),<br>Full text; Figs. 1 to 6<br>& JP 9-66111 A          & EP 847775 A1 | 1-8 |
| A | JP 9-248344 A (Hisamitsu Pharmaceutical Co., Inc.),<br>22 September, 1997 (22.09.97),<br>Full text; Figs. 1 to 8<br>& WO 97/34657 A1          & EP 900576 A1<br>& US 6330471 B1 | 1-8 |

| ☒ Further documents are listed in the continuation of Box C. | ☐ See patent family annex. |
| --- | --- |

| * Special categories of cited documents:<br>"A"  document defining the general state of the art which is not considered to be of particular relevance<br>"E"  earlier document but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |
| --- | --- |

| Date of the actual completion of the international search<br>    30 April, 2003 (30.04.03) | Date of mailing of the international search report<br>    13 May, 2003 (13.05.03) |
| --- | --- |
| Name and mailing address of the ISA/<br>    Japanese Patent Office | Authorized officer |
| Facsimile No. | Telephone No. |

Form PCT/ISA/210 (second sheet) (July 1998)

EP 1 484 084 A1

**INTERNATIONAL SEARCH REPORT**

International application No.

PCT/JP03/00601

C (Continuation). DOCUMENTS CONSIDERED TO BE RELEVANT

| Category* | Citation of document, with indication, where appropriate, of the relevant passages | Relevant to claim No. |
|---|---|---|
| A | JP 10-234366 A  (Hisamitsu Pharmaceutical Co., Inc.), 08 September, 1998 (08.09.98), Full text; Figs. 1 to 9 (Family: none) | 1-8 |

Form PCT/ISA/210 (continuation of second sheet) (July 1998)

10